# DEMANDE DE BREVET EUROPEEN

(11) **EP 0 700 662 A1**
(43) Date de publication de la demande: **13.03.1996**
(21) Numéro de dépôt: 95401939.4
(22) Date de dépôt: 23.08.1995
(51) Int. Cl.: A61B 17/28

(54) **Instrument chirurgical modulaire, notamment de chirurgie coelioscopique**

(30) Priorité: 12.09.1994 FR 9410861
(71) Demandeur: MIKROLAND, F-74250 Fillinges (FR)
(72) Inventeur: Jacquot, Christophe, F-74270 Clarafond (FR)
(74) Mandataire: Lanceplaine, Jean-Claude

(57) **Abrégé**

L'invention a pour objet un instrument chirurgical modulaire, notamment de chirurgie coelioscopique, du type comprenant une poignée (1) comportant deux branches (2, 3) munies chacune d'un anneau de préhension (2a, 3a) et reliées à l'extrémité arrière d'un corps (4), un tube (5) fixé sur l'extrémité avant du corps (4) et une tige (6) traversant le tube et ayant une première extrémité débouchant au niveau de l'extrémité libre du tube (5) et comportant une pince (7) et une seconde extrémité traversant le corps (4) et reliée par un organe de liaison aux branches (2 et 3) pour ouvrir ou fermer la pince (7). Le tube (5) et la tige (6) sont démontables l'un par rapport à l'autre ainsi que du corps (4) et le corps (4) comporte des moyens (20) d'entraînement en rotation du tube (5) et le tube (5) comporte des moyens d'entraînement en rotation et d'immobilisation en translation de la tige (6). Les moyens (20) d'entraînement en rotation du tube (5) sont disposés dans une cassette indépendante et démontable du corps (7).

## Description

La présente invention a pour objet un instrument chirurgical modulaire, notamment de chirurgie coelioscopique.

On sait que la chirurgie coelioscopique consiste à introduire dans l'abdomen, par une courte incision, un instrument chirurgical destiné par exemple à couper des vaisseaux et des artères, à retirer un organe ou encore à retirer des tissus.

Généralement, les instruments pour ce genre de chirurgie se composent d'une poignée de type revolver ou plate comportant deux branches munies chacune d'un anneau de préhension et montées sur un corps.

Au moins une de ces branches est montée pivotante sur le corps et est destinée à commander l'ouverture et la fermeture d'une pince ou d'un ciseau prévu à l'extrémité libre d'une tige fixée sur le corps de l'instrument.

La tige est montée dans un tube et est reliée à une source d'alimentation pour amener le courant à la pince ou au ciseau afin de cautériser les vaisseaux ou les artères.

Mais, les instruments utilisés jusqu'à maintenant présentent des inconvénients importants.

En effet, les différents éléments composant l'instrument, c'est à dire la poignée, le corps, le tube et la tige forment un ensemble monobloc.

Ainsi, chaque outil destiné à un type particulier d'intervention est obligatoirement équipé d'une poignée ce qui augmente sensiblement le coût de chaque instrument.

De plus, ces instruments ne présentent pas toutes les garanties d'hygiène absolument indispensables, étant donné que les différents éléments sont indissociables ce qui pose des problèmes pour la désinfection et l'entretien desdits instruments.

En effet, ces instruments comportent entre les différents éléments des interstices constituant des caches pour les bactéries qui sont très difficiles à atteindre pour effectuer une stérilisation aussi parfaite que possible de ces instruments.

L'invention a pour but d'éviter ces inconvénients en proposant un instrument chirurgical modulaire, notamment de chirurgie coelioscopique, dont tous les éléments sont amovibles et interchangeables ce qui facilite la stérilisation et diminue les risques de contamination.

L'invention a pour objet un instrument chirurgical modulaire, notamment de chirurgie coelioscopique, du type comprenant :
- une poignée comportant deux branches munies chacune d'un anneau de préhension et reliées à l'extrémité arrière d'un corps, au moins l'une desdites branches étant montée pivotante sur ladite extrémité arrière du corps,
- un tube fixé sur l'extrémité avant du corps,
- une tige traversant le tube et ayant, d'une part, une première extrémité débouchant au niveau de l'extrémité libre du tube et comportant une pince ou un ciseau et, d'autre part, une seconde extrémité traversant le corps et reliée par un organe de liaison à la branche pivotante pour ouvrir ou fermer la pince ou le ciseau,
- des moyens de liaison électrique solidaires du corps pour relier électriquement par l'intermédiaire de la tige, la pince ou le ciseau avec une source d'alimentation électrique,

caractérisé en ce que le tube et la tige sont démontables l'un par rapport à l'autre ainsi que du corps, en ce que le corps comporte des moyens d'entraînement en rotation du tube et en ce que ledit tube comporte des moyens d'entraînement en rotation et d'immobilisation en translation de la tige.

Suivant une caractéristique de l'invention, les moyens d'entraînement en rotation sont disposés dans une cassette indépendante et démontable du corps.

Selon encore une autre caractéristique de l'invention, les moyens d'entraînement en rotation du tube sont formés par :
- une molette comportant, sur l'une de ses faces, un axe percé d'un alésage central pour le passage de la tige,
- un capot comportant un alésage central pour le passage, d'une part, de l'axe de la molette et, d'autre part, d'un embout de section cylindrique, fixé à l'extrémité du tube destinée à être montée sur l'extrémité avant du corps, ledit embout étant percé d'un alésage central pour le passage de la tige,
- des moyens d'engrènement de la molette avec l'embout du tube.

D'autres caractéristiques et avantages de l'invention apparaîtront au cours de la description qui va suivre, faite en référence aux dessins annexés, sur lesquels :
- la Fig. 1 est une vue schématique de dessus de l'ensemble de l'instrument chirurgical selon l'invention,
- la Fig. 2 est une vue schématique de côté à plus grande échelle et en coupe longitudinale de la partie centrale de l'instrument chirurgical selon l'invention,
- la Fig. 3 est une vue schématique en coupe selon la ligne 3-3 de la Fig. 2,
- la Fig. 4 est une vue en perspective du corps de l'instrument chirurgical selon l'invention,
- la Fig. 5 est une vue en perspective de la molette des moyens d'entraînement en rotation du tube,
- la Fig. 6 est une vue en perspective du capot des moyens d'entraînement en rotation du tube,
- la Fig. 7 est une vue en perspective de l'embout du tube,

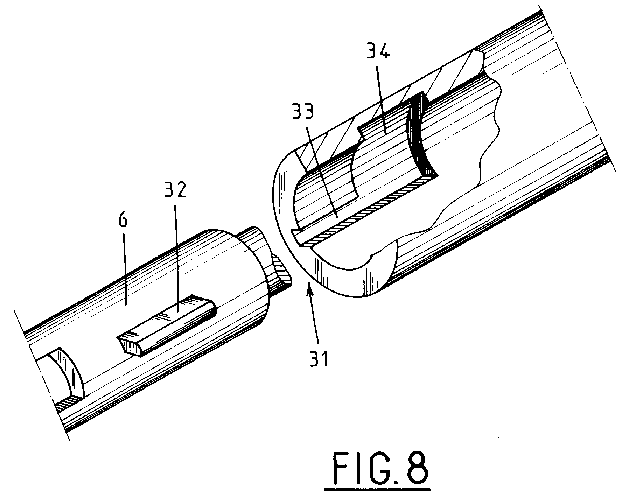

- la Fig. 8 est une vue en perspective de l'extrémité de la tige et du tube,
- la Fig. 9 est une vue schématique en coupe longitudinale de la partie centrale de l'instrument avec la molette en position d'entraînement en rotation du tube,
- la Fig. 10 est une vue schématique de côté en coupe longitudinale d'une variante de l'instrument chirurgical selon l'invention,
- la Fig. 11 est une vue schématique de dessus de l'instrument chirurgical représenté à la Fig.10.

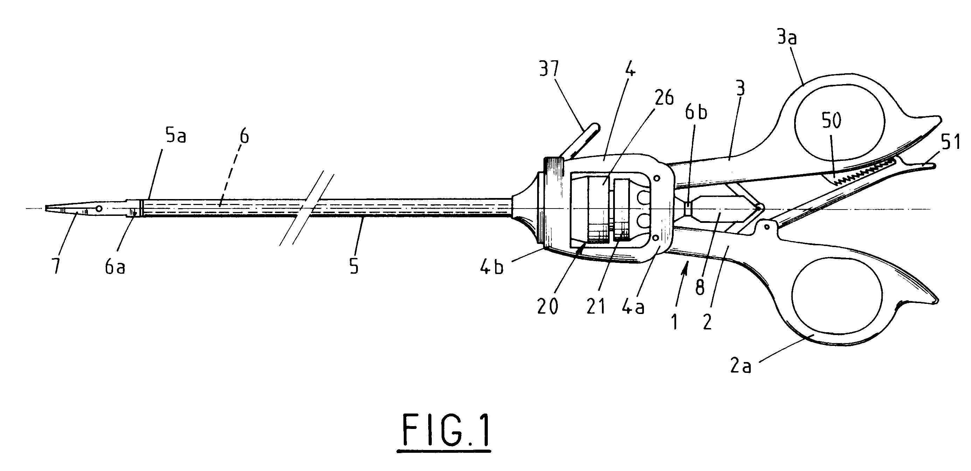

Sur la Fig. 1, on a représenté schématiquement un instrument chirurgical modulaire, notamment de chirurgie coelioscopique qui, comme on le sait, consiste à introduire dans l'abdomen, par une courte incision un instrument chirurgical destiné par exemple à couper des vaisseaux et des artères, à retirer un organe ou encore à retirer des tissus.

L'instrument chirurgical représenté à la Fig. 1 se compose d'une poignée désignée dans son ensemble par la référence 1 comportant deux branches 2 et 3 munies chacune d'un anneau de préhension, respectivement 2a et 3a, destiné au passage des doigts de la main du chirurgien.

La poignée 1 est du type plate comme représenté à la Fig. 1. Cette poignée peut également être du type révolver.

Les branches 2 et 3 sont reliées à l'extrémité arrière 4a d'un corps 4 et au moins l'une desdites branches 2 et 3 est montée pivotante sur ladite extrémité du corps 4.

Les deux branches 2 et 3 peuvent être montées pivotantes sur l'extrémité arrière 4a du corps 4.

L'instrument chirurgical comporte également, d'une part, un tube 5, par exemple en matériau isolant, fixé sur l'extrémité avant 4b du corps 4 et, d'autre part, une tige 6, par exemple en matériau conducteur électriquement, traversant le tube 5 et le corps 4.

La tige 6 a une première extrémité 6a débouchant au niveau de l'extrémité libre 5a du tube 5 et comportant un outil 7 et, d'autre part, une seconde extrémité 6b traversant le corps 4 et reliée par un organe de liaison 8 classique aux branches 2 et 3.

L'outil 7 est constitué par exemple par une pince de préhension ou de dissection ou encore par un ciseau ou une pince de préhension de forte section.

Grâce à l'organe de liaison 8 de la tige 6 avec les branches 2 et 3 de la poignée 1, le chirurgien peut par pivotement des branches 2 et 3 commander l'ouverture ou la fermeture de l'outil 7, c'est à dire des mors de la pince ou des lames des ciseaux.

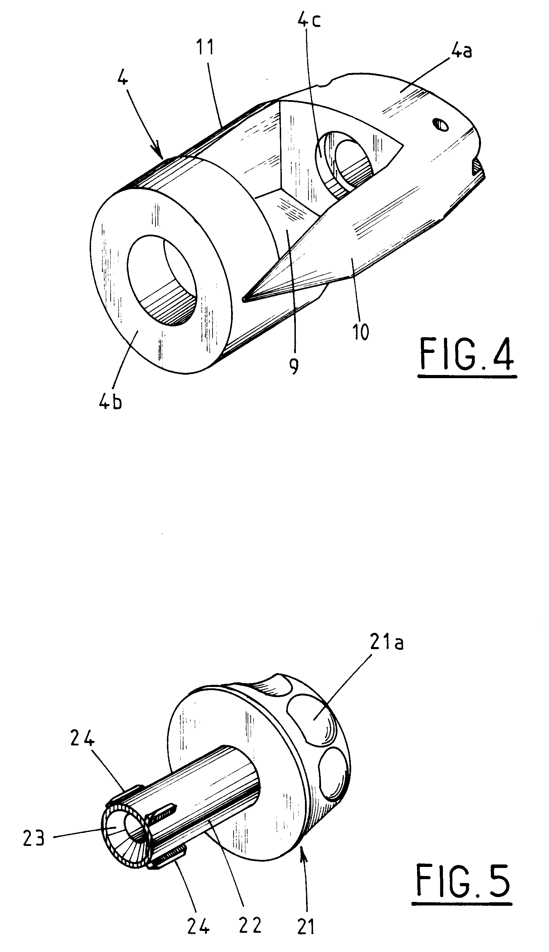

Comme représenté à la Fig. 4, le corps 4 de l'instrument chirurgical comporte un évidement 9 et deux branches latérales 10 et 11 qui relient les extrémités arrière et avant respectivement 4a et 4b dudit corps 4.

Le tube 5 et la tige 6 sont démontables l'un par rapport à l'autre ainsi que du corps 4 et le corps 4 comporte des moyens 20 d'entraînement en rotation du tube 5.

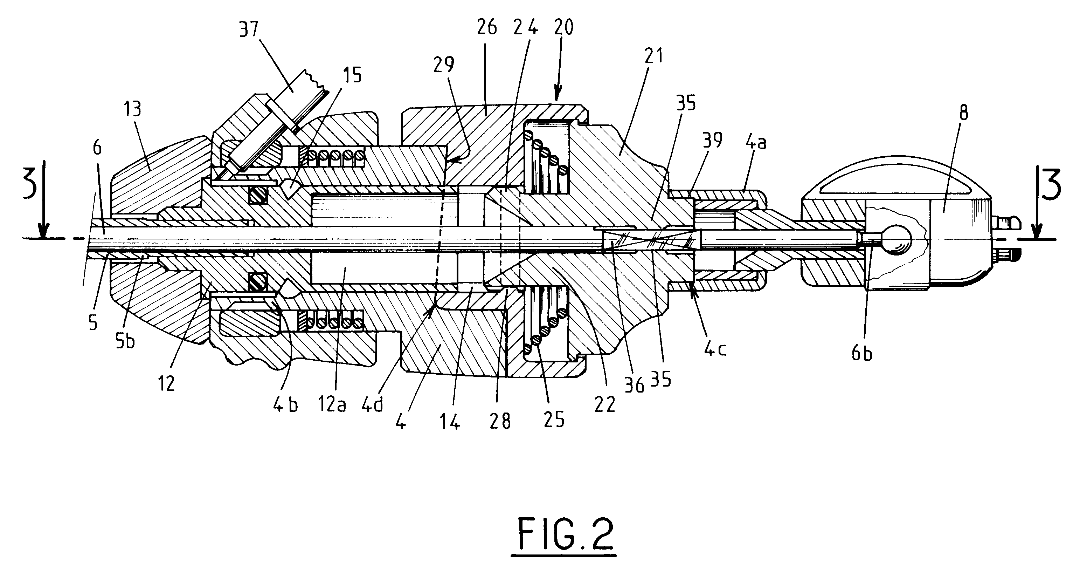

Ainsi que représenté sur les Figs. 2, 3 et 7, le tube 5 comporte à son extrémité 5b destinée à être montée sur l'extrémité avant 4b du corps 4, un embout 12 de section cylindrique.

L'extrémité 5b du tube 5 comporte également, juste avant l'embout 12, une bague de protection 13.

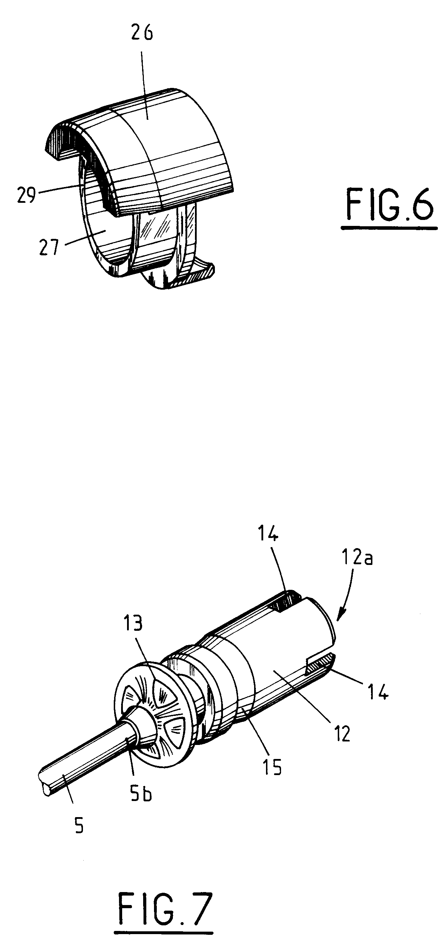

Les moyens 20 d'entraînement en rotation du tube 5 sont formés d'une molette 21 représentée à la Fig. 5, d'un capot 26 représenté à la Fig. 6 et de moyens d'engrènement de la molette 21 avec l'embout 12 du tube 5.

Ainsi que représenté à la Fig. 5, la molette 21 comporte sur l'une de ses faces un axe 22 percé d'un alésage centrale 23 pour le passage de la tige 6.

La face externe du corps de la molette 21 est pourvue d'empreintes 21a pour le positionnement de l'extrémité d'un doigt de la main du chirurgien de façon à entraîner la molette 21 en rotation.

Comme représenté sur les Figs. 2, 3 et 6, le capot 26 comporte un alésage central 27 pour le passage, d'une part, de l'axe 22 de la molette 21 et, d'autre part, de l'embout 12 du tube 5. La molette 21 est par exemple encliquetée dans le capot 26.

L'embout 12 est également percé d'un alésage central 12a pour le passage de la tige 6.

Les moyens d'engrènement de la molette 21 avec l'embout 12 sont débrayables et sont formés par des dents 24 ménagées sur la face externe de l'axe 22 de la molette 21 et par des rainures 14 ménagées sur l'extrémité libre de l'embout 12 du tube 5.

Les dents 24 et les rainures 14 sont par exemple au nombre de quatre.

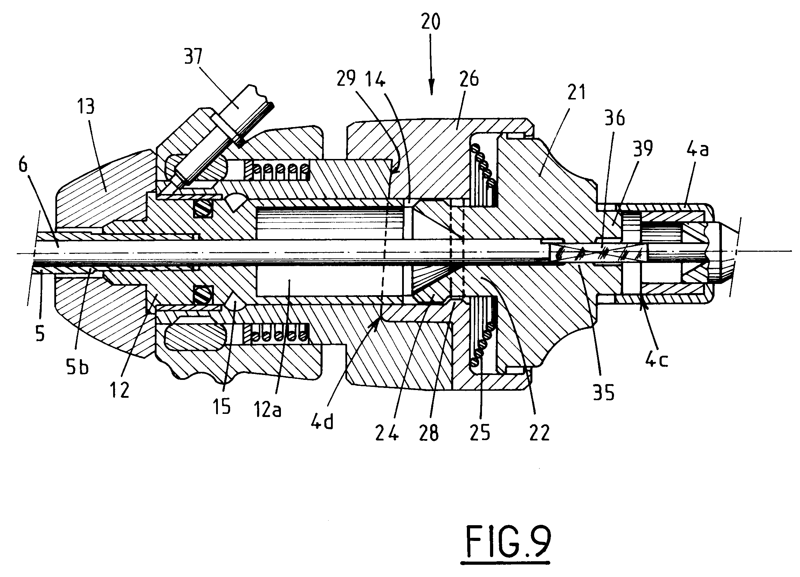

La molette 21 est déplaçable longitudinalement entre, d'une part, une première position dans laquelle cette molette est immobilisée en rotation par des moyens de blocage comme représenté à la Fig. 2 et, d'autre part, une seconde position dans laquelle ladite molette 21 est libre en rotation et les moyens d'engrènement constitués par les dents 24 et les rainures 14 sont en prise comme représentées à la Fig. 9.

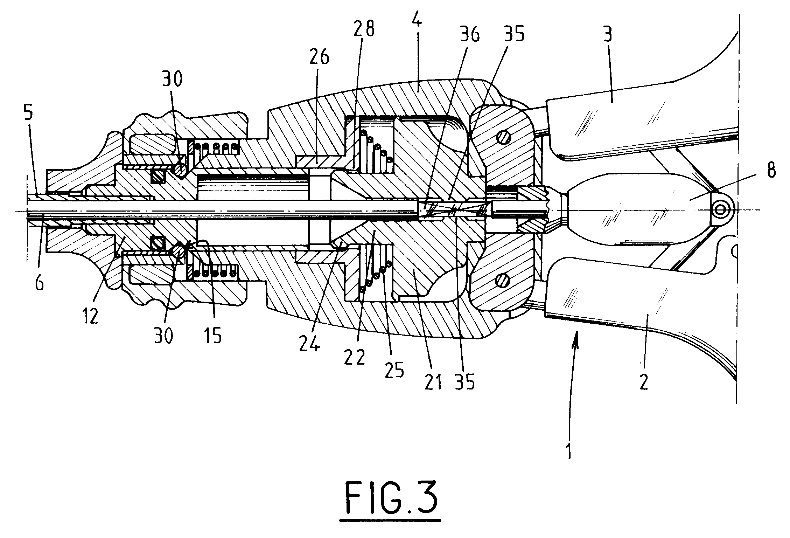

La molette 21 comporte un organe de rappel de ladite molette dans la première position, constitué par exemple par un ressort 25 interposé entre cette molette 21 et le capot 26 comme cela est représenté sur les Figs. 2 et 3.

Les moyens de blocage en rotation de la molette 21 dans la première position représentée à la Fig. 2, sont formés par des nervures 28 ménagées à l'extrémité de l'alésage 27 du capot 26 située en regard de la molette 21.

Le capot 26 est immobilisé en rotation et les nervures 28 sont destinées à coopérer avec les dents 24 de l'axe 22 de la molette 21 pour immobiliser également en rotation la molette 21 dans la première position.

Les moyens 20 d'entraînement en rotation du tube 5, c'est à dire la molette 21, le capot 26 et le ressort 25 sont disposés dans une cassette indépendante et démontable du corps 4.

Pour cela, et comme représenté à la Fig. 2, la molette 21 comporte, sur sa face opposée à celle munie de l'axe 22, un bossage 39 destiné à pénétrer dans un logement 4c formé à l'intérieur de l'évidement 9 au niveau de l'extrémité arrière 4a du corps 4 et le capot 26 comporte, sur sa face opposée à celle en regard de ladite molette 21, un plan incliné 29 destiné a coopérer avec le fond de l'évidement 9 qui comporte également un plan incliné 4d de pente complémentaire au plan incliné 29.

Pour démonter la cassette formée par la molette 21 et le capot 26, il suffit de comprimer le ressort 25 par déplacement longitudinal de la molette 21 de façon à sortir le bossage 39 du logement 4c et de soulever la cassette.

La molette 21 et le capot 26 sont facilement séparables l'un de l'autre ce qui facilite la désinfection et la stérilisation de ces éléments.

Lorsque l'embout 12 du tube 5 est en position dans l'alésage 27 du capot 26, la molette 21 et le capot 26 ne sont pas démontables du corps 4.

Pour empêcher un démontage intempestif du tube 5 du corps 4, l'extrémité avant 4b de ce corps 4 est pourvue de moyens de blocage en translation dudit tube 5.

Les moyens de blocage en translation sont formés par deux axes parallèles 30 opposés et montés dans l'alésage de l'extrémité avant 4b du corps 4. Ces axes 30 sont disposés transversalement par rapport au tube 5 et sont destinés à pénétrer dans des fentes en V 15 ménagées sur l'embout 12 du tube 5.

Au moment du montage du tube 5 sur le corps 4, l'embout 12 glisse dans l'alésage de l'extrémité avant 4b du corps 4 jusqu'à ce que les axes 30 s'encliquettent dans les fentes 15 de façon à immobiliser le tube 5 et empêcher le démontage de la molette 21 et du capot 26.

Le tube 5 comporte également les moyens d'entraînement en rotation et d'immobilisation en translation de la tige 6.

Ces moyens d'entraînement et d'immobilisation en rotation de la tige 6 par le tube 5 sont formés par un système d'assemblage à baïonnette prévu à l'extrémité libre du tube 5 et désigné dans son ensemble 31.

De manière classique, ce système d'assemblage à baïonnette 31 est formé par des ergots 32 portés par la tige 6 et destinés à pénétrer dans une première rainure 33 parallèle à l'axe du tube 5 et, par rotation de la tige 6, dans une seconde rainure 34 perpendiculaire à l'axe dudit tube 5.

Ainsi, lorsque les ergots 32 sont en position dans les rainures 34 ménagées sur la face interne du tube 5, la tige 6 est immobilisée en rotation et en translation par rapport au tube 5.

De plus, la molette 21 comporte des moyens de solidarisation en rotation de la tige 6 avec ladite molette 21 pour empêcher le démontage intempestif de cette tige 6 lorsque le tube 5 est en position dans le corps 4.

Comme représenté sur les Figs. 2 et 3, ces moyens de solidarisation en rotation de la tige 6 avec la molette 21 sont formés par un orifice 35 de section carrée ménagé dans l'alésage central 23 de ladite molette 21 et par un carré 36 formé sur la tige 6.

Ainsi, lorsque la tige 6 est placée en position dans le corps 4, l'orifice 35 coopère avec le carré 36 pour empêcher la rotation de cette tige 6 indépendamment de la rotation de la molette 21.

De plus, l'instrument chirurgical comporte des moyens de liaison électrique solidaires du corps 4 pour relier électriquement, par l'intermédiaire de la tige 6, l'outil 7 avec une source d'alimentation électrique, non représentée, pour cautériser les vaisseaux ou les artères.

Ces moyens de liaison électrique sont constitués par exemple par un plot 37 fixé sur le corps 4, comme représenté à la Fig. 2. Dans ce cas, l'embout 12 du tube 5 est réalisé en matériau conducteur.

Pour tourner l'outil 7, le chirurgien déplace la molette 21 vers l'avant de l'instrument, à l'encontre du ressort 25, ce qui a pour effet de désengager les dents 24 des nervures 28 (Fig. 9). Ensuite, il suffit au chirurgien de tourner la molette 21 dans un sens ou dans l'autre tout en maintenant la pression vers l'avant ce qui provoque la rotation du tube 5, de la tige 6 et de l'outil 7 par l'intermédiaire du système à baïonnette 31.

Dès que le chirurgien relâche la molette 21, celle-ci est de nouveau verrouillée, empêchant ainsi toute rotation intempestive de l'outil 7.

Dans le cas où le chirurgien souhaite pouvoir tourner l'outil 7 sans avoir à déplacer la molette 21, par exemple pour des ciseaux, il dispose d'une autre cassette et il lui suffit de changer de cassette.

Dans cette seconde cassette qui permet au chirurgien de pouvoir tourner la molette 21 sans avoir à la déplacer vers l'avant de l'instrument, les dents 24 sont plus courtes et sont de ce fait désengagées des nervures 28 ce qui permet de rendre la molette 21 libre en rotation.

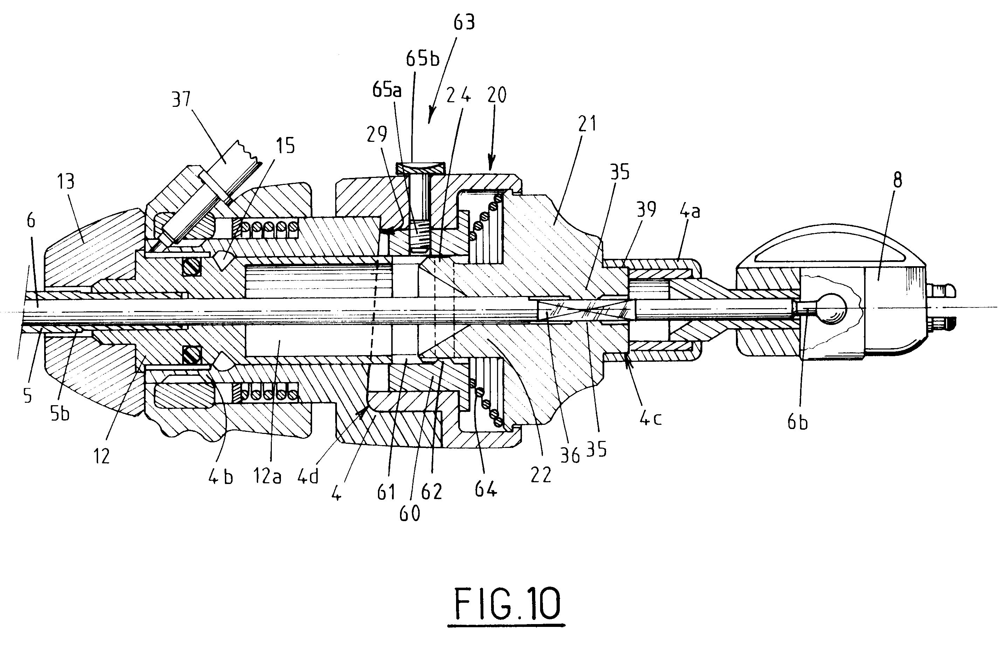

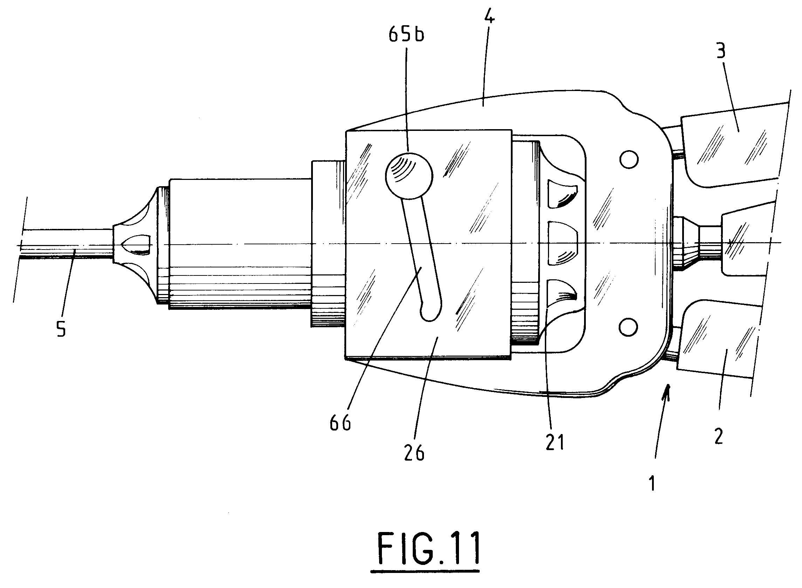

En se rapportant aux Fig.10 et 11, on va décrire une variante de l'instrument chirurgical selon l'invention.

Sur ces figures, les éléments communs au précédent mode de réalisation sont désignés par les mêmes références.

Selon ce mode de réalisation, la molette 21 est fixe longitudinalement et coopère avec des moyens débrayables de blocage en rotation de ladite molette 21.

Ces moyens débrayables de blocage en rotation de la molette 21 sont formés par une bague 60 comportant un alésage central 61 pour le passage de l'axe 22 de la molette 21 et cet alésage central 61 est muni de nervures 62.

La bague 60 est déplaçable longitudinalement dans le capot 26 par un organe de commande 63 entre, d'une part, une première position dans laquelle la molette 21 est immobilisée en rotation par engrênement des nervures 62 avec les dents 24 de l'axe 22 de cette molette 21 et, d'autre part, une seconde position dans laquelle ladite molette 21 est libre en rotation par désengagement des nervures 62 avec lesdites dents 24.

Un organe de rappel 64 de la bague 60 dans la première position est interposé entre ladite bague 60 et la molette 21. Cet organe de rappel 64 est formé par exemple par un ressort.

L'organe de commande 63 est constitué par une tige verticale 65 dont l'extrémité inférieure 65a est solidaire de la bague 60 et dont l'extrémité supérieure 65b débouche sur la face externe du capot et comporte par exemple un bouton. La tige verticale 65 est déplaçable transversalement à l'axe de la bague 60 dans une lumière 66 ménagée dans le capot 26. Cette lumière 66 est inclinée par rapport à un plan perpendiculaire à l'axe de la bague 60 comme représenté à la Fig.11 pour déplacer longitudinalement la bague 60 vers l'extrémité arrière 4a du corps 4 entre les deux positions indiquées précédemment.

Enfin, l'une des branches de la poignée 1, comme par exemple la branche 3 comporte une crémaillère 50 et l'autre desdites branches, comme par exemple la branche 2 comporte une gachette 51 destinée à coopérer avec cette crémaillère 50 pour bloquer les branches 2 et 3 dans une position souhaitée.

L'instrument chirurgical selon l'invention présente de nombreux avantages par rapport aux instruments utilisés jusqu'à présent.

Tout d'abord, le tube et la tige sont démontables l'un par rapport à l'autre et du corps de l'instrument, si bien qu'une même poignée peut être utilisée successivement pour une pince de préhension ou de dissection ou encore pour des ciseaux.

L'outil disposé à l'extrémité de la tige peut également être constitué par une pince de préhension de forte section isolée par rapport au plot de liaison électrique. Dans ce cas, l'embout du tube est réalisé en matériau isolant.

De plus, l'ensemble du mécanisme de commande de la rotation du tube et de la tige forme une cassette interchangeable et démontable du corps et les différents éléments composant ce mécanisme sont également démontables.

De ce fait, la stérilisation des différentes pièces est facilitée et les règles d'hygiène sont ainsi respectées.

## Revendications

**1 -** Instrument chirurgical modulaire, notamment de chirurgie coelioscopique, du type comprenant:
- une poignée (1) comportant deux branches (2, 3) munies chacune d'un anneau de préhension (2a, 3a) et reliées à l'extrémité arrière (4a) d'un corps (4), au moins l'une desdites branches (2, 3) étant montée pivotante sur ladite extrémité arrière (4a) du corps (4),
- un tube (5) fixé sur l'extrémité avant (4a) du corps (4),
- une tige (6) traversant le tube et ayant, d'une part, une première extrémité (6a) débouchant au niveau de l'extrémité libre (5a) du tube (5) et comportant une pince (7) ou des ciseaux et, d'autre part, une seconde extrémité (6b) traversant le corps (4) et reliée par un organe de liaison (8) à la poignée pivotante pour ouvrir ou fermer la pince (7) ou les ciseaux,
- des moyens (37) de liaison électrique solidaires du corps (4) pour relier électriquement, par l'intermédiaire de la tige (6), la pince (7) ou les ciseaux avec une source d'alimentation électrique,
caractérisé en ce que le tube (5) et la tige (6) sont démontables l'un par rapport à l'autre ainsi que du corps (4), en ce que le corps (4) comporte des moyens (20) d'entraînement en rotation du tube (5) et en ce que ledit tube (5) comporte des moyens (31) d'entraînement en rotation et d'immobilisation en translation de la tige (6).

**2 -** Instrument chirurgical selon la revendication 1, caractérisé en ce que les moyens (20) d'entraînement en rotation du tube (5) sont disposés dans une cassette indépendante et démontable du corps (4).

**3 -** Instrument chirurgical selon les revendications 1 et 2, caractérisé en ce que les moyens (20) d'entraînement en rotation du tube (5) sont formés par:
- une molette (21) comportant, sur l'une de ses faces, un axe (22) percé d'un alésage central (23) pour le passage de la tige (6),
- un capot (26) comportant un alésage central (27) pour le passage, d'une part, de l'axe (22) de la molette (21) et, d'autre part, d'un embout (12) de section cylindrique fixé à l'extrémité (5b) du tube (5) et destiné à être monté sur l'extrémité (4b) avant du corps (4), ledit embout (12) étant percé d'un alésage central (12a) pour le passage de la tige (6),
- des moyens d'engrènement de la molette (21) avec l'embout (12) du tube (5).

**4 -** Instrument chirurgical selon la revendication 3, caractérisé en ce que les moyens d'engrènement de la molette (21) avec l'embout (12) du tube (5) sont débrayables.

**5 -** Instrument chirurgical selon les revendications 3 et 4, caractérisé en ce que la molette (21) est déplaçable longitudinalement entre, d'une part, une première position dans laquelle cette molette (21) est immobilisée en rotation par des moyens (28) de blocage, et d'autre part, une seconde position dans laquelle ladite molette (21) est libre en rotation et les moyens d'engrènement sont en prise.

**6 -** Instrument chirurgical selon l'une des revendications 3 à 5, caractérisé en ce que les moyens d'engrènement sont formés par des dents (24) ménagées sur la face externe de l'axe (22) de la molette (21) et par des rainures (14) ménagées sur l'extrémité libre de l'embout (12) du tube (5).

**7 -** Instrument chirurgical selon la revendication 5,-caractérisé en ce qu'un organe de rappel (25) de la molette (21) dans la première position, formé par exemple par un ressort, est interposé entre ladite molette (21) et le capot (26).

**8 -** Instrument chirurgical selon les revendications 5 et 6, caractérisé en ce que les moyens de blocage en rotation de la molette (21) sont formés par des nervures (28) ménagées à l'extrémité de l'alésage (27) du capot (26) située en regard de la molette (21), lesdites nervures (28) étant destinées à coopérer avec les dents (24) de l'axe (22) de la molette (21) dans la première position de ladite molette (21).

**9 -** Instrument chirurgical selon la revendication 4, caractérisé en ce que la molette (21) est fixe longitudinalement et coopère avec des moyens débrayables de blocage en rotation de la molette (21), formés par une bague (60) comportant un alésage central (61) pour le passage de l'axe (22) de la molette (21) et muni de nervures (62), ladite bague (60) étant déplaçable longitudinalement dans le capot (26) par un organe de commande (63) entre, d'une part, une première position dans laquelle la molette (21) est immobilisée en rotation par engrênement des nervures (62) avec les dents (24) de l'axe (22) de cette molette (21) et, d'autre part, une seconde position dans laquelle ladite molette (21) est libre en rotation par désengagement des nervures (62) avec les dents (24).

**10 -** Instrument chirurgical selon la revendication 9, caractérisé en ce qu'un organe de rappel (64) de la bague (60) dans la première position, formé par exemple par un ressort, est interposé entre ladite bague (60) et la molette (21).

**11.** Instrument chirurgical selon la revendication 9, caractérisé en ce que l'organe de commande (63) est formé par une tige verticale (65) dont l'extrémité inférieure 65(a) est solidaire de la bague (60) et dont l'extrémité supérieure 65(b) débouche sur la face externe du capot (26), ladite tige verticale (65) étant déplaçable transversalement à l'axe de la bague (60) dans une lumière (66) inclinée et ménagée dans le capot (26) pour déplacer longitudinalement ladite bague (60).

**12 -** Instrument chirurgical selon la revendication 1, caractérisé en ce que les moyens d'entraînement et d'immobilisation en rotation de la tige (6) par le tube (5) sont formés par un système d'assemblage à baïonnette (31) prévu à l'extrémité libre du tube (5).

**13 -** Instrument chirurgical selon l'une des revendications précédentes, caractérisé en ce que la molette (21) comporte des moyens de solidarisation en rotation de la tige (6) avec ladite molette (21).

**14 -** Instrument chirurgical selon la revendication 13, caractérisé en ce que les moyens de solidarisation én rotation de la tige (6) avec la molette (21) sont formés par des méplats (35) ménagés dans l'alésage central (23) de la molette (21) et destinés à coopérer avec un carré (36) formé sur ladite tige (6).

**15 -** Instrument chirurgical selon l'une des revendications précédentes, caractérisé en ce que l'extrémité avant (4b) du corps (4) est munie de moyens de blocage en translation du tube (5).

**16 -** Instrument chirurgical selon la revendication 15, caractérisé en ce que les moyens de blocage en translation du tube (5) sont formés par un anneau élastique (30) disposé dans l'alésage du corps (4) et destiné à s'encliqueter dans un gorge (15) ménagée sur l'embout (12) du tube (5).

**17 -** Instrument chirurgical selon l'une des revendications précédentes, caractérisé en ce que la molette (21) comporte, sur sa face opposée à celle munie de l'axe (22), un bossage (39) destiné à pénétrer dans un logement (4c) formé au niveau de l'extrémité arrière (4a) du corps (4) et le capot (26) comporte, sur sa face opposée à celle en regard de ladite molette (21), un bossage (29) destiné à pénétrer dans un logement (4d) formé au niveau de l'extrémité avant (4b) du corps (4).

**18 -** Instrument chirurgical selon la revendication 1, caractérisé en ce que l'une des branches (2, 3) comporte une crémaillère (50) et l'autre desdites branches (2, 3) comporte une gachette (51) destinée à coopérer avec cette crémaillère (50) pour bloquer les branches (2, 3) de la poignée (1) dans une position souhaitée.
